# EUROPEAN PATENT SPECIFICATION

(11) **EP 4 571 642 B1**
(45) Date of publication and mention of the grant of the patent: **19.08.2026**
(21) Application number: 23307178.6
(22) Date of filing: 12.12.2023
(51) Int. Cl.: G06T 7/11, A61B 6/03, G06V 10/82

(54) **MEASUREMENT OF HUMAN BODY TISSUE**
MESSUNG VON MENSCHLICHEM KÖRPERGEWEBE
MESURE DE TISSU HUMAIN DU CORPS

(43) Date of publication of application: 18.06.2025
(73) Proprietor: Dassault Systèmes, 78140 Vélizy-Villacoublay (FR)
(72) Inventor: Roullet, Agathe, 78946 Vélizy-Villacoublay Cedex (FR); Ball, Arthur, 78946 Vélizy-Villacoublay Cedex (FR)
(74) Representative: Bandpay & Greuter

(56) References cited:
- SHU-YEN WAN ET AL: "Extraction of the Hepatic Vasculature in Rats Using 3-D Micro-CT Images", IEEE TRANSACTIONS ON MEDICAL IMAGING, IEEE, USA, vol. 19, no. 9, 1 September 2000 (2000-09-01), XP011036022, ISSN: 0278-0062
- FAN MING ET AL: "Computer-aided detection of mass in digital breast tomosynthesis using a faster region-based convolutional neural network", METHODS, ACADEMIC PRESS, NL, vol. 166, 13 February 2019 (2019-02-13), pages 103 - 111, XP085782110, ISSN: 1046-2023, [retrieved on 20190213], DOI: 10.1016/J.YMETH.2019.02.010
- CAI JINZHENG ET AL: "Lesion-Harvester: Iteratively Mining Unlabeled Lesions and Hard-Negative Examples at Scale", IEEE TRANSACTIONS ON MEDICAL IMAGING, IEEE, USA, vol. 40, no. 1, 7 September 2020 (2020-09-07), pages 59 - 70, XP011827741, ISSN: 0278-0062, [retrieved on 20201229], DOI: 10.1109/TMI.2020.3022034

## Description

### TECHNICAL FIELD

The disclosure relates to the field of computer programs and systems, and more specifically to a method, system and program for measuring a human body tissue from a set of medical images.

### BACKGROUND

Medical imaging is becoming of greater importance in clinical trials. In oncology, 95% of studies use medical imaging to evaluate the efficacy of treatments measured by the time to progression of the tumor disease (e.g., Progression-Free Survival), or the objective response rate (ORR).

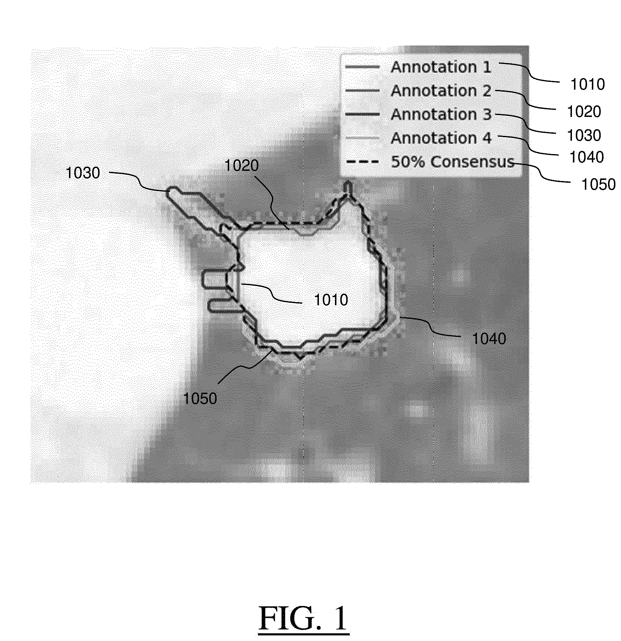

A number of systems and programs exist for analyzing medical images such as CT-Scans. For example, 3DS MEDIDATA RAVE Imaging provides an IT infrastructure for the collection of imaging data for centralized image review based on international best practice recommendations: the RECIST (Response Evaluation Criteria in Solid Tumours) criterion. The document *"*Eisenhauer, E. A., et. al, New response evaluation criteria in solid tumours: revised RECIST guideline (version 1.1). European journal of cancer, 45(2), 228-247, (2009*)"* is the revised guideline of the RECIST criterion that define a standard approach to solid tumor measurement and definitions for objective assessment of change in tumor size for use in adult and pediatric cancer clinical trials. 3DS MEDIDATA RAVE Imaging relies on the expertise of radiologists. The process of measuring a solid tumor is manual, repetitive, tedious, and associated with measurement errors and variability between experts. FIG. 1 illustrates an example of annotations 1010-1040 on an identified lesion using the RECIST process, and a 50% consensus 1050. The annotations 1010-1040 have multiple differences due to interradiologists' variability, who are in charge of manually revising medical images for identifying and measuring target lesions in the human body as well for identifying new lesions. In practice, the radiologist choses one of the image of the set with their best practice, and they perform the measurement of the tissue on that image.

Approaches have been developed for predicting 2D segmentations of the tumors on CT-scans. One of these approach has been described in the paper Yan, K., et al, MULAN: Multitask Universal Lesion Analysis Network for Joint Lesion Detection, Tagging, and Segmentation," MICCAI 2019, that relies on a maskrcnn model specialized to deal with CT-scans in order to predict 2D segmentations of the tumors. All CT-scans slices are annotated with all tumors that have been detected on them. But those measurements are of limited help to the radiologist, as he must annotate a singular slice per lesion to comply with RECIST standard ; in other words, an intervention of the radiologist is still needed for interpretating the CT-scans. In addition, this approach requires one specialized model per organ for performance reasons.

In a related paper Cai, J., Harrison, A. P., Zheng, Y., Yan, K., Huo, Y., Xiao, J., ... & Lu, L. (2020). Lesion-harvester: Iteratively mining unlabeled lesions and hard-negative examples at scale. IEEE Transactions on Medical Imaging, 40(1), 59-70, is described a method to detect relevant tumor 3D bounding boxes but it does not provide automatic segmentation nor RECIST measurements. Again, intervention of the radiologist is still needed for interpretating the CT-scans.

Also known in the art are:
- Shu-Yen Wan et. al., Extraction of the hepatic vasculature in rats using 3-D micro-CT images, which discloses an automatic procedure for extracting and representing a vascular tree;
- Fan Ming et. al., Computer-aided detection of mass in digital breast tomosynthesis using a faster region-based convolutional neural network, which discloses a detection architecture of convolution neural network with a region proposal network (RPN) used for each slice to generate region proposals with a mass likelihood score; and
- Cai Jinzheng et. al. Lesion-Harvester: Iteratively Mining Unlabeled Lesions and Hard-Negative Examples at Scale, which discloses a system to harvest missing annotations from lesion datasets.

Hence, the main problems with the above-discussed methods are that the accuracy of the interpretation of the CT-scans lies on the expertise of the radiologist, and also the process is manual, repetitive, tedious.

Within this context, there is still a need for an improved method for measuring a human body tissue from a set of medical images representing the human body tissue.

### SUMMARY

The invention is set out in the appended set of claims.

### BRIEF DESCRIPTION OF THE DRAWINGS

Non-limiting examples will now be described in reference to the accompanying drawings, where:
- FIG. 1 shows an example of the prior art;
- FIG. 2 shows a flowchart of an example of the method;
- FIG. 3 shows an example of the system; and
- FIG.s 4 to 8 illustrate examples of the method.

### DETAILED DESCRIPTION

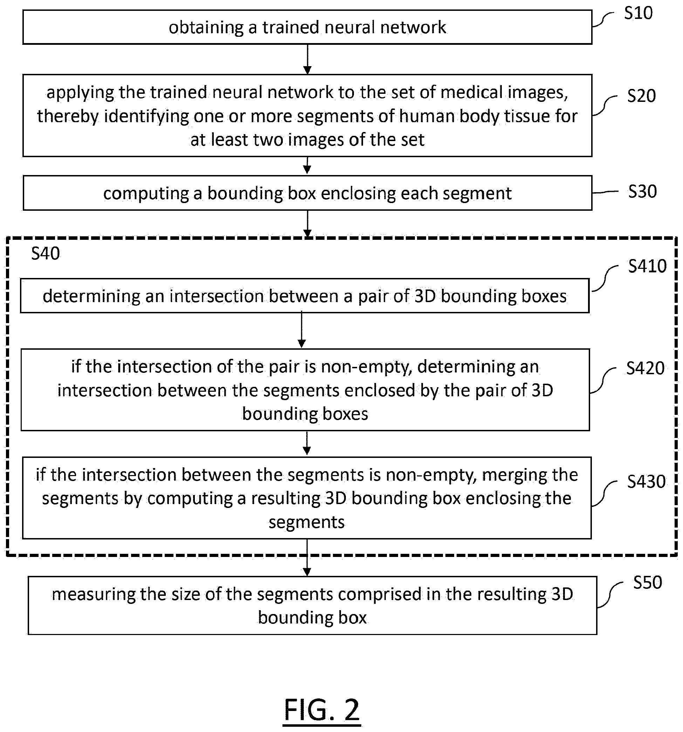

With reference to the flowchart of FIG. 2, it is proposed a computer-implemented method for measuring a human body tissue from a set of medical images representing the human body tissue. The method (also called "measurement method") comprises obtaining S10 a trained neural network configured for outputting segments of human body tissue from the images of the set. The method also comprises applying S20 the trained neural network to the set of medical images. The method thereby identifies one or more segments of human body tissue for at least two images of the set. The method also computes S30 a bounding box enclosing each segment that have been identified. The method also determines S410 an intersection between a pair of bounding boxes. If the intersection of the pair is non-empty, the method determines S420 an intersection between the segments enclosed by the pair of bounding boxes. If the intersection between the segments is non-empty, the method merges S430 the segments by computing a resulting bounding box enclosing the segments. The method also measures S50 the size of the segments comprised in the resulting bounding box.

Such a method improves the measurement of a human body tissue on a set of medical images representing the human body tissue.

Notably, the method obtains an accurate measurement of the human body tissue thanks to the way the segments are determined. Indeed, thanks to the segments being comprised in the resulting bounding box, the method accurately determines anatomically correct representation of the human body tissue, as the segments comprised in the resulting bounding box are ensured to belong to the same human body tissue. For example, the human body tissue may appear as a single segment on one image of the set of images while another image may show multiple segments of that tissue. By construction, the resulting bounding box comprises the segments of the two images of the set which represent the same tissue. The method thus eliminates user bias when determining the appropriate segments for performing measurements of the human body tissue.

As said before, the method arrives to an accurate determination of an anatomically correct representation of the human body tissue thanks to the computation of the resulting bounding box. This is all thanks to the specific steps performed by the method. First, the method leverages from the paradigm of neural networks for identifying the one or more segments of the human body tissue for at least two images of the set. The method computes a bounding box enclosing each segment and determines an intersection between a pair of bounding boxes. The method thus performs a coarse detection of segments (comprised in the pair of bounding boxes) which are likely to belong to the same human body tissue. Such coarse detection is computationally efficient and particularly fast. If the intersection of the pair of bounding boxes is non-empty, the method determines the intersection between the segments enclosed by the pair of bounding boxes. In other words, the method performs a fine-grained determination segments belonging to the same human body tissue. This fine-grained determination allows the method to avoid false positives that may have been retained on the coarse detection, e.g., bounding boxes which intersect but do not have intersecting segments. The segments merged by computation of the resulting bounding box thus comprise segments belonging to the same human body tissue.

It is further provided a method for identifying (also called "identification method") the evolution of a human body tissue. The identification method comprises obtaining a current set of medical images representing human body tissue of a patient. Obtaining the current set of medical images may comprise retrieving such set from a non-volatile storage, or retrieving the set from a network.

The identification method also comprises using the measurement method for obtaining a current measurement of the size of the segments. In other words, the measurement method takes as input the current set of medical images and outputs a measurement of the size of the segments comprised in resulting bounding boxes. The current set of medical images is obtained at any time.

The identification method also comprises obtaining a measurement obtained from a past set of medical images representing the human body tissue of the patient. In other words, the measurement method takes as input the past set of medical images and outputs a measurement of the size of the segments comprised in resulting bounding boxes. The past set of medical image represents the same human body tissue as the current measurement. The past set of medical images is taken at a time preceding the time (and thus the time in which the past set and the current set are obtained is different, e.g., by some hours, or days or even months) in which the current set of medical images is obtained.

The identification method also comprises computing the difference between the current measurement and the past measurement. The difference may be a simple subtraction. The identification method thereby identifies the evolution of the human body tissue. Indeed, the identification indicates the evolution of the human body tissue according to the change (as represented by the difference) of dimension of the segment from which the measurement is determined.

The methods disclosed herein are computer-implemented. This means that steps (or substantially all the steps) of the method(s) are executed by at least one computer, or any system alike. Thus, steps of the method(s) are performed by the computer, possibly fully automatically, or, semi-automatically. In examples, the triggering of at least some of the steps of the method(s) may be performed through user-computer interaction. The level of user-computer interaction required may depend on the level of automatism foreseen and put in balance with the need to implement user's wishes. In examples, this level may be user-defined and/or predefined.

A typical example of computer-implementation of a method is to perform the method with a system adapted for this purpose. The system comprise a processor coupled to a memory and may comprise a graphical user interface (GUI). The memory has recorded thereon a computer program comprising instructions for performing the method. The memory may also store a database. The memory is any hardware adapted for such storage, possibly comprising several physical distinct parts (e.g. one for the program, and possibly one for the database).

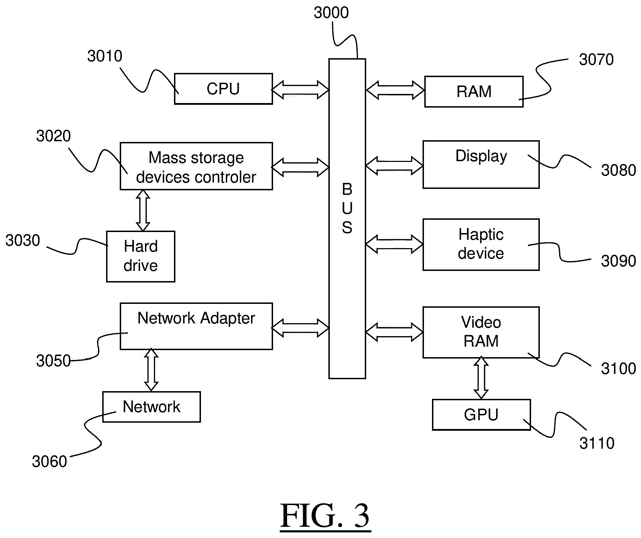

FIG. 3 shows an example of the system, wherein the system is a client computer system, e.g. a workstation of a user.

The client computer of the example comprises a central processing unit (CPU) 3010 connected to an internal communication BUS 3000, a random access memory (RAM) 3070 also connected to the BUS. The client computer is further provided with a graphical processing unit (GPU) 3110 which is associated with a video random access memory 3100 connected to the BUS. Video RAM 3100 is also known in the art as frame buffer. A mass storage device controller 3020 manages accesses to a mass memory device, such as hard drive 3030. Mass memory devices suitable for tangibly embodying computer program instructions and data include all forms of nonvolatile memory, including by way of example semiconductor memory devices, such as EPROM, EEPROM, and flash memory devices; magnetic disks such as internal hard disks and removable disks; magneto-optical disks. Any of the foregoing may be supplemented by, or incorporated in, specially designed ASICs (application-specific integrated circuits). A network adapter 3050 manages accesses to a network 3060. The client computer may also include a haptic device 3090 such as cursor control device, a keyboard or the like. A cursor control device is used in the client computer to permit the user to selectively position a cursor at any desired location on display 3080. In addition, the cursor control device allows the user to select various commands, and input control signals. The cursor control device includes a number of signal generation devices for input control signals to system. Typically, a cursor control device may be a mouse, the button of the mouse being used to generate the signals. Alternatively or additionally, the client computer system may comprise a sensitive pad, and/or a sensitive screen.

The computer program may comprise instructions executable by a computer, the instructions comprising means for causing the above system to perform the method. The program may be recordable on any data storage medium, including the memory of the system. The program may for example be implemented in digital electronic circuitry, or in computer hardware, firmware, software, or in combinations of them. The program may be implemented as an apparatus, for example a product tangibly embodied in a machine-readable storage device for execution by a programmable processor. Method steps may be performed by a programmable processor executing a program of instructions to perform functions of the method by operating on input data and generating output. The processor may thus be programmable and coupled to receive data and instructions from, and to transmit data and instructions to, a data storage system, at least one input device, and at least one output device. The application program may be implemented in a high-level procedural or object-oriented programming language, or in assembly or machine language if desired. In any case, the language may be a compiled or interpreted language. The program may be a full installation program or an update program. Application of the program on the system results in any case in instructions for performing the method. The computer program may alternatively be stored and executed on a server of a cloud computing environment, the server being in communication across a network with one or more clients. In such a case a processing unit executes the instructions comprised by the program, thereby causing the method to be performed on the cloud computing environment.

The set of medical images may be formed by a data structure comprising (e.g., through storage in a physical memory such as a hard drive) a plurality (e.g. two or more) of medical images.

In examples, the set of medical images may comprise a set of CT-SCAN images, or a set of MRI images, or a set of PET-scan images or a set of ultrasound images. A CT-scan (Computed Tomography Scan) is a specific type of medical images produced by sending X-rays through the human body. The signal is read and analyzed to reconstruct a dense volume of the body. Practically, the volume is split into slices whose normal direction is feet to head. An MRI (Magnetic Resonance Imaging) is a specific type of medical images technique that uses a magnetic field and computergenerated radio waves to create detailed images of the organs and tissues in the body. PET-scan (Positron Emission Tomography) is an imaging test that can help reveal the metabolic or biochemical function of your tissues and organs. The PET scan uses a radioactive drug called a tracer to show both typical and atypical metabolic activity. Ultrasound imaging (sonography) uses high-frequency sound waves and images are produced based on the reflection of the waves off of the body structures. The strength (amplitude) of the sound signal and the time it takes for the wave to travel through the body provide the information necessary to produce an image.

The medical images of the set represent a human body tissue. A tissue is a group of cells, in close proximity, organized to perform one or more specific functions, as known in the art. The human body tissue may thus correspond to a lesion in an organ (such as the liver, heart or bones) or may correspond to a tissue corresponding to an aneurism or may be an organ (e.g., a portion of the organ or all of the organ). The lesion may be an anomaly in a human body without presuming the pathogenicity in the human body tissue. In examples, in the context of oncology the term lesion may be used for referring to a tumor.

The set of medical images may be images ordered according to a reference axis. In other words, each image may comprise a representation of the human body tissue viewed from a view (e.g., orthogonal with respect to the reference axis) at a position of the reference axis. The reference axis is set by convention, e.g., a standard z axis along the human body portion comprising the human body tissue, in which case each view comprises a view on the x-y plane (that is, an axial or transversal view of the human body tissue). The images of the set may comprise information of the position of the images with respect to the reference axis, that is, the position of the reference axis in which the representation of the human body tissue is viewed. The images of the set may be ordered according to such information. In examples, the images of the set may be evenly distributed, that is, the images sample the portion human body evenly (with a same separation between two consecutive images of the ordered set) along the reference axis. For example, the images may be separated along the reference axis by 1mm or more.

The method obtains S10 the trained neural network. This means that the computerized system performing the method can access directly the neural network (e.g. the neural network runs onto the computerized system or is executed by the computerized system) or indirectly the neural network (e.g. the computerized system can provide an input to the neural network and obtain the output of the neural network obtained for the input). The trained neural network is configured for outputting segments of human body tissue from the images of the set. In other words, the trained neural network performs a segmentation of the objects in an image. Image segmentation (also referred to as object segmentation) groups pixels that belong to the same object by categorizing each pixel value of an image to a particular class, as known in the art.

The neural network may be a function comprising a collection of connected nodes, also called "neurons". Each neuron receives an input and outputs a result to other neurons connected to it. The neurons and the connections linking each of them have weight values, which are adjusted via a training. In the context of the present disclosure, by "trained neural network", it is meant that weights of the neural network are definitively saved after performing a learning/training on a dataset. The dataset may comprise sets of medical images each comprising one or more annotated segments of human body tissue. The dataset may comprise medical images from the DeepLession dataset, for example as set forth in the document Yan K, Wang X, Lu L, Summers RM, DeepLesion: automated mining of large-scale lesion annotations and universal lesion detection with deep learning, J Med Imaging (Bellingham), 2018.

The trained neural network may be a deep convolutional neural network such as a Mask R-CNN neural network set forth in the document Kaiming He, Georgia Gkioxari, Piotr Dollár, Ross Girshick Mask R-CNN, arXiv:1703.06870.

The method applies S20 the trained neural network to the set of medical images. In other words the medical images of the set are provided in input of the trained neural network. The trained neural network thereby identifies one or more segments of human body tissue for at least two images of the set; the one or more identified segments of human body tissue are outputted by the trained neural network. In other words, the method processes the set of medical images as input of the trained neural network, and outputs the one or more segments of human body tissue according to the weight values of the trained neural network. As known per se from the field of machine-learning, the processing of an input by a neural network includes applying operations to the input, the operations being defined by data including the weight values.

A segment may be any collection of pixels corresponding to a region of a corresponding medical image (that is, the medical image in which the trained neural network is applied). The pixels of the segment may comprise a color indicative of a presence of the human body tissue. A segment can therefore be referred to as 2D segment as the trained neural network performs 2D segmentation.

The method computes S30 a bounding box enclosing each segment that have been identified by the trained neural network. By "bounding box" it is meant any geometrical shape in any dimension such as 2 or more, e.g., three dimensions, for example a, two-dimensional rectangle, a rectangular parallelepiped in three dimensions. In the case of three dimensions the bounding box can be called bounding volume. The bounding box encloses each segment. In other words, the bounding box comprises (or encloses) the pixels of the segment indicative of the presence of the human body tissue. The method may take into account the spatial position of the segment. For example, the method may convert the segments into 3D segments, wherein the 3D position of the segments may be the position relative to the x-y plane of their respective medical image and to the z-axis defined by the reference axis. The method may determine the size of the bounding box from the difference between the positions of the pair of images along the reference axis, or from the difference between two consecutive pair of images in the case in which the pair of images are evenly distributed.

The method determines S410 an intersection between pair of bounding boxes. The determination of the intersection may include computing the intersection of at least one point (or line or surface) belonging to a respective bounding box of the pair. The method may retain the position of each bounding box according to the position of the segment in the respective image, and with respect to the reference axis for determining the intersection.

The intersection of the pair of bounding boxes may be empty or non-empty. An intersection is empty when there is no geometrical intersection or crossing/overlap between two elements (e.g., points, lines or surface) each corresponding to a respective bounding box of the pair. An intersection is non-empty if there is a geometrical intersection or crossing/overlap between two elements of each respective bounding box of the pair.

If the intersection S410 of the pair of bounding boxes is non-empty, the method determines S420 an intersection between the segments enclosed by the pair of bounding boxes. The determination of the intersection between the segments may include determining the intersection of at least a pair of points (or lines or surfaces) belonging to a respective segment enclosed by a bounding box of the pair.

If the intersection S420 between the segments is non-empty, the method merges S430 the segments by computing a resulting bounding box enclosing the segments. In other words, the method creates a new bounding box which encloses the segments. The method may discard the pair of bounding boxes enclosing the segments upon creating the resulting bounding box.

In examples, the method may further comprise, if the intersection between the segments is empty, maintaining the pair of bounding boxes enclosing each segment. In other words, the segments are not merged when the intersection is empty. Thereby, the method ensures that the segments are not merged unnecessarily, for example when the segments do not correspond to the same human body tissue, or when the segments are too far away.

The method measures S50 the size of the segments comprised in the resulting bounding box. The method may measure a diameter or transversal section of each segment.

In example, measuring the size of the segments may further comprise selecting the segment comprised in the resulting bounding box having the longest diameter, that is the longest distance. The method may determine the longest diameter by computing all distances between lines connecting pairs of points of a contour of the segment, and keeping the line having maximum distance as the longest diameter. The method may also determine a short diameter. The short diameter (or short distance) may be the determined from the lines connecting two points of the contour and which is orthogonal to the line corresponding to the longest diameter. The short diameter may correspond to the line having the longest distance among the lines orthogonal to the line corresponding to the longest diameter.

The measurement may be based on such longest diameter. For example, the measure S50 may consist of the length of such longest diameter. This is the case where the segment is representative of human body tissue such as non-lymph nodes lesions.

Alternatively, in the case where the segment is representative of human body tissue such as a lymph nodes, the measurement consists of the largest segment of the segment which is orthogonal to such longest diameter. In other words, the short diameter.

Hence, different types of measures may be provided and the selection of a type of measure may depend on the type of human tissue represented on the image, for example a RECIST diameter for tumoral lesions, the measurement of a diameter of an aneurism or the size of an organ. Measuring a human body tissue thus amounts to obtaining at least one distance between two points on the representation of the human body tissue. The distance may be an Euclidian distance.

As the method applies the trained neural network to the set of medical images, the one or more segments of human body tissue are obtained in a particularly fast and accurate manner.

The set of medical images representing the human body tissue may be seen as a sampling of the human body tissue along a reference axis. The computation of the bounding box allows to determine which segments belong to a same body tissue thanks to the spatial position of the bounding boxes. In other words, the bounding boxes provide a 3D sense to the one or more segments so as to allow the determination of which segments belong to a same body tissue according to the distribution of the images along the reference axis.

For identifying which segments belong to the same body tissue, the method determines the intersection between the pair of bounding boxes. Thus, the method detects as early as possible when segments do not intersect. The intersection between the pair of bounding boxes may be seen as a coarse approximation. The method moves to a fine-grained computation: after determining the intersection between the pair of bounding boxes, when determining that such intersection is non-empty, the method then determines the intersection between the pair segments which discards wrongly associated segments.

When the method merges the two segments by computing the resulting bounding box, as refining the determination of the human body tissue for its measurement. The resulting bounding box encompasses the intersecting segments, and thus the method may measure the size of the segments so belonging to the same body tissue. This indeed improves the accuracy of the measurement.

The method further comprises, prior to determining the intersection between the segments, computing 2D bounding boxes for each identified segment from the at least two images of the set. Each 2D bounding box encloses an identified segment. The 2D bounding box may be a 2D parallelepiped (e.g. a square, a rectangle) having the minimum area so as to enclose the identified segment. The method may retain the 2D positions of the segment so as to position the corresponding 2D bounding box, e.g., a position such as the center of the 2D bounding box.

The trained neural network may also be configured for outputting 2D bounding boxes enclosing the segments of human body tissue. Computing the 2D bounding boxes may be carried out by the trained neural network. In other words, the trained neural network may be applied to the set of medical images. The trained neural network thereby outputs a respective 2D bounding box for each respective segment. For example, the trained neural network may be a deep convolutional neural network such as a Mask R-CNN neural network that outputs segments and bounding boxes for each segment.

The method also comprises determining an intersection between at least two bounding boxes among the computed 2D bounding boxes. In other words, the method determines the intersection in 2D coordinates of the computed 2D bounding boxes. For example, determining the intersection between the 2D bounding boxes may include determining the intersection of at least a pair of points (or lines) belonging to a respective 2D bounding box.

The determination of the intersection between the segments may be only performed for segments for which the intersection between 2D bounding boxes is non-empty. That is, the method does not pursue the determination of the intersection between the segments when the intersection between the 2D bounding boxes is empty. This allows to perform a more accurate detection of intersection than using the 3D bounding boxes while being relatively fast, compared to the detection of the intersection between segments.

The method may further comprise iteratively determining S40 the intersection S410 over pairs of bounding boxes by repeating the determining S410, S420 and the merging S430 for each remaining pair of bounding boxes. That is, the method may repeat the determination of the intersection S410 followed by the determination of the intersection between the segments enclosed by the pair of segments S420 and followed by the merging S430 of the segments (if the intersection between the segments is non-empty). As the method computes a bounding box for each segment, the method may proceed, in another iteration, to perform the determination S410 between the pair of other bounding boxes after merging the segments 5430, or upon determining that either the intersection of the pair of bounding boxes is empty, or upon determining that the intersection between the segments is empty.

The method may perform the measurement S50 after the iterative determination S40 is completed, for example upon determining that there are no longer pairs of intersecting bounding boxes having a non-empty intersection.

The method may further comprise obtaining a distance between the images comprising the segments enclosed by the pair. The distance between the images may be obtained from the order of the images according to a reference axis. In other words, the distance may correspond to the difference in the position of each image with respect to the reference axis. The determination S420 of the intersection between the segments enclosed by the pair may be only performed when the distance between the images comprising the segments is below a predetermined threshold.

The trained neural network may be also configured for outputting tags identifying segments of human body tissue. The tag may be any piece of information indicative of the human body tissue. The determination of the intersection over pairs of bounding boxes is performed for segments having the same tag. In other words, the determination is performed for segments representative of the same body tissue, as captured by the set of images. This ensures that the measurement of the human body tissue is anatomically correct.

Determining the intersection between the segments may comprises obtaining a mask for each segment. A mask may be a binary image consisting of zero and nonzero values. Pixels corresponding to the segment may have a predetermined value, e.g., 1, whereas other pixels (for instance, pixels corresponding to other portions of the human body) have a value of zero. Determining the intersection may also comprise computing the intersection between the obtained masks. The computation of the intersection may comprise determining a pixel-wise intersection. For example, two binary masks may be determined to intersect if at least one pixel at a position of a given mask (at an x-y position of the mask) has the same value as a pixel of the given mask at the same position. The merging of the segments may be only performed for segments for which the intersection between the obtained masks is non-empty. In other words, the bounding boxes may be maintained if the intersection is empty. This results in a finer intersection determination, which is thereby more accurate.

Examples are now discussed with reference to FIG.s 4 to 9.

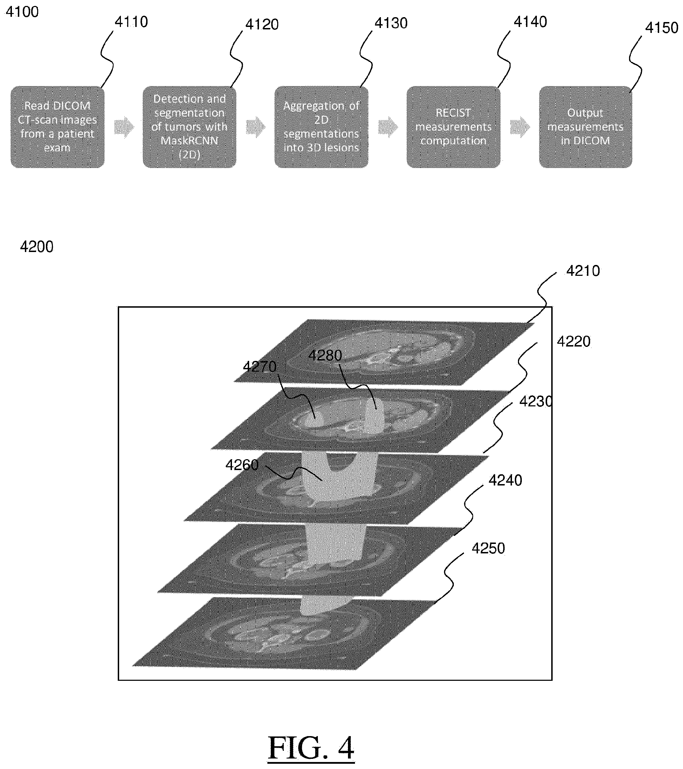

FIG. 4 shows an example of a pipeline for implementing the method.

The pipeline 4100 may comprise obtaining the set of medical images by reading, for example DICOM CT-scan images or NIFTI images taken from a patient (for instance during examination) 4110. DICOM refers to a file format dedicated to store medical images. The DICOM format may comprise several modalities (eg: CT, RMI, US), some of such modalities may comprise text reports or description of geometrical objects. The method may be exemplified by stages 4120 to 4140, wherein the step 4120 corresponds to the application S20 of the trained neural network to the set of medical images, the steps 4130 and 4140 refers to steps S30 to S50 of the method. The result of the measurements may be output in the DICOM format 4150.

Interestingly, the method steps S10 to S50 may be carried by a computerized system that is interfaced between the device producing the images (e.g. a CT-Scanner), and the device that takes the images as inputs to display them to the radiologist, e.g., a viewer. No adaptation of the two devices is needed. In other words, the computer system that carries out the method steps S10 to S50 may be contemplated as a software plugin connected at the output of the device producing the images and at the input of the display device.

The set of medical images 4200 of the example comprises five medical images 4210 to 4250. Each medical image provides a 2D representation of a part of the human body at, for example a transversal view or an axial view. The medical images 4210-4250 sample the body tissue 4260 (shown as a mass for the sake of illustration only). The images of the set are ordered according to the standard z axis (not represented). The medical image 4220 illustrates a case where it represents two body tissues 4270 and 4280 which actually belong to the same body tissue 4260. The determination of the intersections between the pairs of bounding boxes (e.g., the bounding boxes that respectively enclose the segments on the images 4220 and 4230), the determination S420 and the merging S440 resulting to a bounding box enclosing the segments of the images (e.g., the segments of the images 4220 and 4230) allow to determine that the two body tissues 4270 and 4280 belong to the same body tissue 4260.

An example of the method comprising the iterative determination of the intersection over pairs of bounding boxes is now discussed.

The method converts the segmentations into individual lesions, being understood that the method applies to any human body tissue. Then, the method iterates on all pairs of lesions to see whether they intersect. If they do, they are merged together; otherwise, we proceed with other lesions. After the method has finished comparing all lesions altogether, the method verifies that newly merged lesions do not have new intersections. Therefore, the method loops back on all lesions. Such aggregation process finishes when no more merging occurs. At that point, the method outputs the lesions.

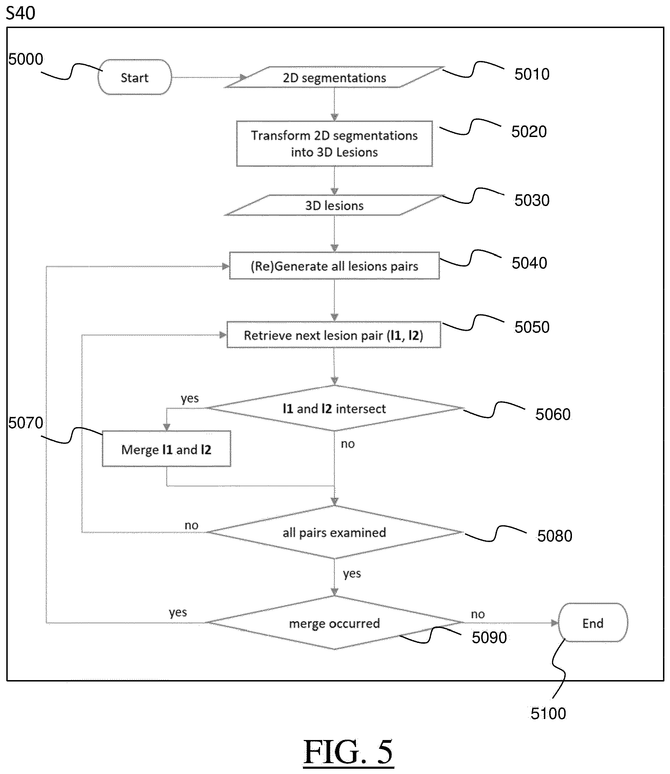

Reference is made to FIG. 5, illustrating an example of the iterative determination S40.

The iterative determination S40 starts 5000 and selects obtained segments, e.g., as 2D segmentations 5010 identified from the application S20 of the trained neural network to the set of medical images. The method may transform 5020 the 2D segmentations into 3D segments (also called "3D lesions") so as to give the segments a 3D position 5030. Here the term lesions is used in reference to medical images obtained for measuring potential increase or decrease of a lesion. However, any type of human body tissue may be used. The transformation of a 2D segment into a 3D segment aims at providing an artificial thickness to the 2D segment. In examples, the thickness are obtained by computing a bounding box encompassing the 2D segment, the bounding box having a thickness at least equal to the distance between two consecutive images of the set of images; being understood that this distance is the same of each pair of consecutive images of the set of medical images. The thickness of the bounding box may be equals to the space between two consecutive images of the set of images. For instance, if the resolution of the imaging device producing the set of medical images is of 1mm (that is the distance according to the Z axis is 1mm between two consecutive images of the set -the slices-), the thickness of the bounding boxes for obtaining the 3D lesions is 1mm. Interestingly, computing (obtaining) 3D bounding boxes is cost efficient in term of computing resources and memory. Experiences have shown that using the resolution of the imaging device as thickness of the bounding boxes provide the best results.

The iteration may continue with a step of generation of all 3D segments (or 3D lesions) pairs 5040, which defines segments which may be obtained from the application of the trained neural network or merged after an iteration.

The method may retrieve a pair of segments (or "lesion pairs" l1, l2) 5050 at a first iteration (or a next lesion pair in a subsequent iteration). The method may determine 5060 if the segments intersect by performing the determination of the intersection 5410 followed by the determination of the intersection between the pair of segments S420. If the segments intersect, the method may proceed 5070 to merging S430 the segments l1 and l2 by computing the resulting bounding box. If the segments do not intersect at S420, the method maintains the pair of bounding boxes enclosing each segment. The method may determine 5080 if all of the pairs from which the bounding boxes have been generated intersect, by determining if there is a non-empty intersection between pairs of resulting bounding boxes. If there is 5090 a non-empty intersection, the method may repeat the step 5040 and following steps. If there are no non-empty intersections, the method determines that the merge between all segments has not occurred 5090, and the method terminates 5100 the iteration 540.

An example of the detection of intersection between segments is now discussed.

Reference is made to FIG. 5, illustrating an example of the determination of the intersection between the pair of bounding boxes, followed by the determination of the intersection between 2D bounding boxes, followed by the determination of the intersection between the segments enclosed by the pair of bounding boxes.

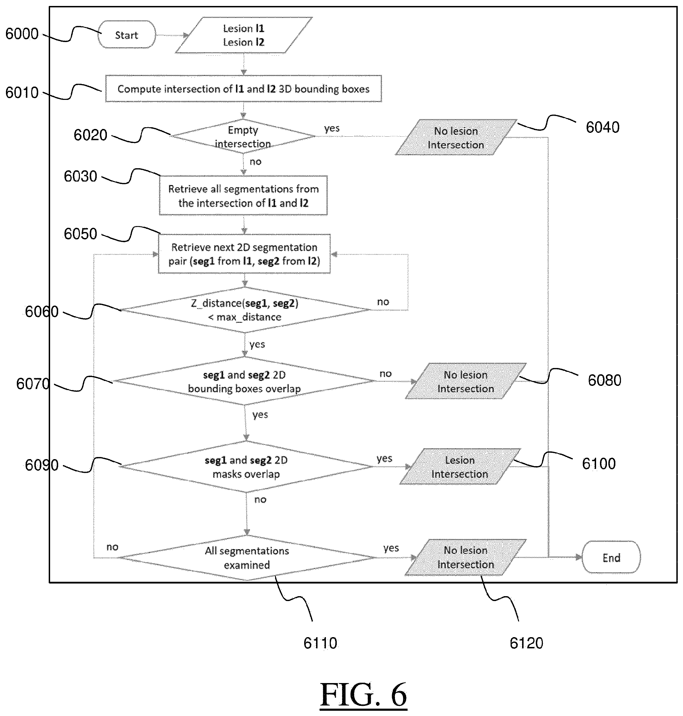

The example of FIG. 6 starts with two 3D segments (also called "3D lesions" discussed in reference to FIG. 5) l1 and l2 6000. The method determines the intersection between a pair of bounding boxes 6010. If the intersection 6020 is empty, the method determines that there is no lesion intersection 6040 and the process terminates. If the intersection 6020 of the pair is non-empty, the method retrieves the segments comprised in the intersecting bounding boxes 6030. The method determines if the segments comprised in the intersecting bounding boxes are separated from the reference axis (in this case the z-axis) below a predetermined threshold *max_distance* 6060, for example by comparing information on the separation of the corresponding images along the reference axis. The *max_distance* may be selected according to the resolution of the scan that produces the images. The *max_distance* may be selected so that it is at the most equals to the space between two consecutive images of the set of medical images. The predetermined threshold *max_distance* 6060 may be selected by performing an exploration of hyperparameters. Alternatively, the predetermined threshold *max_distance* 6060 may be selected as a function of an expected size of a lesion, for example 15mm or more.

In the event in which the separation is below the predetermined threshold, the method computes 2D bounding boxes for each identified segment from the at least two images of the set. The method determines an intersection 6070 between at least two among the computed 2D bounding boxes, e.g., by determining if the 2D bounding boxes overlap. If the intersection between the computed 2D bounding boxes is empty, the method determines that there is no lesion intersection 6080 and the process terminates. In the event where the intersection between the computed 2D bounding boxes is non-empty, the method determines an intersection between the segments 6090 enclosed by the pair of bounding boxes, for example by obtaining a mask for each segment, and determining if the corresponding masks intersect. If the intersection between the masks of the segments is non-empty, the method merges the segments by computing a resulting bounding box enclosing the segments. When two segments are to be merged, a new segment is created that encompasses segmentations from both original lesions. The 3D bounding box is re-computed accordingly. Former lesions are then discarded.

The method determines if all of the segments have been examined 6110. If no, the method returns to step 6050. Else, the method terminates. It is to be understood that the mask of a segment is computed/obtained as known in the art. For example, Mask R-CNN generates in output accurate segmentation mask for each segment.

Hence, the method detects as early as possible when lesions do not intersect. The method goes from coarse approximation to fine-grained computation as the method moves forward towards computing the intersection between mask: first, the method looks at 3D bounding boxes, then method compares 2D bounding boxes of each segmentations pair and finally compares 2D masks.

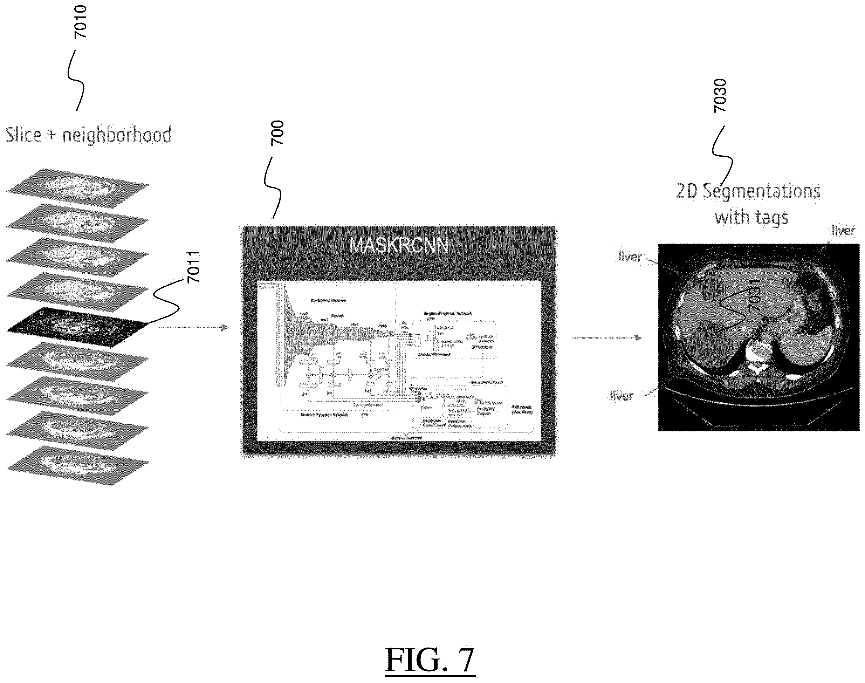

FIG. 7 illustrates the pre-trained neural network.

The method may use a pre-trained 2D deep convolutional neural network (Mask R-CNN) 7020 that has been trained to process CT-scan images 7010.

The network takes a slice image 7011 and its 3D context as input and outputs zero or more detected 2D lesions 7030, for example from the liver 7031. Each detected 2D lesion is then either filtered out or stacked together with other close detected lesions, to make a 3D lesion. The method outputs a RECIST measurement per detected 3D lesion.

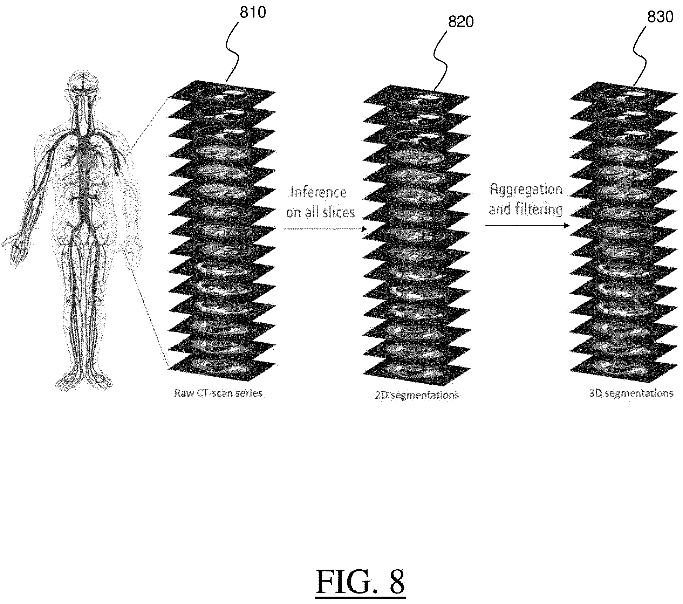

FIG. 8 illustrates the inference of measurements on RAW CT-scans. The method may produce 3D segments accompanied with measurements 820, or stack them together as 3D lesions 830.

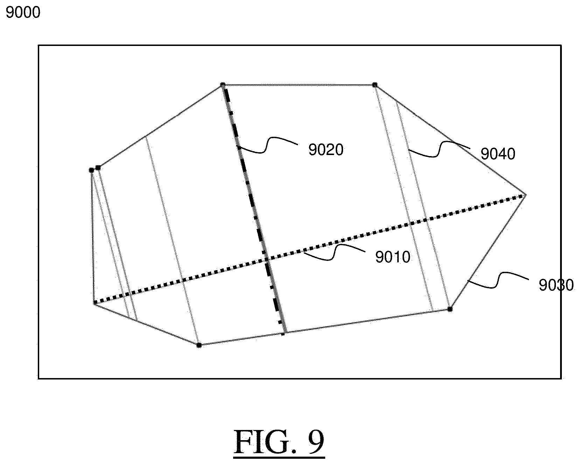

The computation of RECIST measurements is now discussed. Reference is made to FIG. 9. Illustrating the computed RECIST measurement on a segment 9000.

The method localizes two specific diameters for a segment of interest: the long diameter 9010 and the short diameter 9020. Each segment must be measured at most once so the method makes sure selected segment for determining the measurement is the one where the diameters are made maximal. For this, the method measures the diameters of all the slices composing a segment and keeps only the maximum on as the long diameter.

To compute the diameters, the method sets up the following geometric methodology: for the long diameter 9010, the method computes all distances for all pairs of points of the contour and keeps the maximum one. For the short diameter 9020 the method goes through all points of the contour of the segment 9030 and considers the lines 9040 passing through this point and orthogonal to the long diameter 9010 that has been just computed. The method measures the segment that lies inside the contour and keeps the maximum one, which is the line 9020.

## Claims

1. A computer-implemented method for measuring a human body tissue from a set of medical images representing the human body tissue, wherein the images are ordered according to a reference axis, the method comprising:
- obtaining (S10) a trained neural network configured for outputting 2D segments of human body tissue from the medical images of the set;
- applying (S20) the trained neural network to the set of medical images, thereby identifying one or more 2D segments of human body tissue for at least two images of the set;
- computing (S30) a 3D bounding box enclosing each 2D segment, the 3D bounding box having a thickness at least equal to the distance between two consecutive images of the set of images;
- determining (S410) an intersection between a pair of 3D bounding boxes;
- if the intersection of the pair is non-empty, identifying the segments comprised in the intersecting bounding boxes, computing 2D bounding boxes for each identified segment from the at least two images of the set, each 2D bounding box enclosing an identified segment, and determining an intersection between at least two among the computed 2D bounding boxes;
- if the intersection of the pair is non-empty, determining (S420) an intersection between the segments enclosed by the pair of 3D bounding boxes; wherein the determination of the intersection between the segments is only performed for segments for which the intersection between the 2D bounding boxes is non- empty.
- if the intersection between the segments is non-empty, merging (S430) the segments by computing a resulting 3D bounding box enclosing the segments; and
- measuring (S50) the size of the segments comprised in the resulting 3D bounding box.

2. The method of claim 1 further comprising iteratively determining (S40) the intersection (S410) over pairs of bounding boxes by repeating the determining (S420) and the merging (S430) for each remaining pair of bounding boxes.

3. The method of claims 1 to 2, further comprising, if the intersection between the segments is empty, maintaining the pair of bounding boxes enclosing each segment.

4. The method of any one of claims 1 to 3, further comprising obtaining a distance between the images comprising the segments enclosed by the pair, the determination (S420) of the intersection between the segments enclosed by the pair being only performed when the distance between the images comprising the segments is below a predetermined threshold.

5. The method of any one of claims 1 to 4, wherein the trained neural network is also configured for outputting tags identifying segments of human body tissue, and wherein the determination of the intersection over pairs of bounding boxes is performed for segments having the same tag.

6. The method of any one of claims 1 to 5, wherein determining the intersection between the segments comprises:
- obtaining a mask for each segment; and
- computing the intersection between the obtained masks, the merging of the segments being only performed for segments for which the intersection between the obtained masks is non-empty.

7. The method of any one of claims 2 to 6 wherein the trained neural network is also configured for outputting 2D bounding boxes enclosing the segments of human body tissue, and wherein computing the 2D bounding boxes is carried out by the trained neural network.

8. The method of any one of claims 1 to 7, wherein measuring the size of the segments further comprises selecting the segment comprised in the resulting bounding box having the longest diameter.

9. The method of any one of claims 1 to 8, wherein the set of medical images comprises a set of CT-SCAN images, a set of MRI images, PET-scan images or ultrasound images.

10. The method of any one of claims 1 to 9, wherein the human body tissue represented by the set of medical images corresponds to a lesion in an organ, or tissue of an aneurism or an organ.

11. A method for identifying the evolution of a human body tissue, comprising:
- obtaining a current set of medical images representing human body tissue of a patient;
- using the method of any one of claims 1 to 10 for obtaining a current measurement of the size of the segments;
- obtaining a measurement obtained from a past set of medical images representing the human body tissue of the patient;
- computing the difference between the current measurement and the past measurement, thereby identifying the evolution of the human body tissue.

12. A computer program comprising instructions which, when the program is executed by a computer, cause the computer to carry out the method of any of claims 1 to 10 and/or the method of claim 11.

13. A computer readable storage medium having recorded thereon the computer program of claim 12.

14. A system comprising a processor coupled to a memory, the memory having recorded thereon the computer program of claim 12.

## Patentansprüche

1. Computerimplementiertes Verfahren zum Messen eines menschlichen Körpergewebes aus einem Satz medizinischer Bilder, die das menschliche Körpergewebe darstellen, wobei die Bilder gemäß einer Referenzachse geordnet sind, das Verfahren umfassend:
- Erlangen (S10) eines trainierten neuronalen Netzes, das zum Ausgeben von 2D-Segmenten von menschlichem Körpergewebe aus den medizinischen Bildern des Satzes konfiguriert ist;
- Anwenden (S20) des trainierten neuronalen Netzes auf den Satz medizinischer Bilder, wodurch ein oder mehrere 2D-Segmente von menschlichem Körpergewebe für mindestens zwei Bilder des Satzes identifiziert werden;
- Berechnen (S30) einer 3D-Umrahmungsbox, die jedes 2D-Segment umschließt, wobei die 3D-Umrahmungsbox eine Stärke aufweist, die mindestens dem Abstand zwischen zwei aufeinanderfolgenden Bildern des Satzes von Bildern entspricht;
- Bestimmen (S410) einer Schnittmenge zwischen einem Paar von 3D-Umrahmungsboxen;
- wenn die Schnittmenge des Paares nicht leer ist, Identifizieren der in den sich schneidenden Umrahmungsboxen enthaltenen Segmente, Berechnen von 2D-Umrahmungsboxen für jedes identifizierte Segment aus den mindestens zwei Bildern des Satzes, wobei jede 2D-Umrahmungsbox ein identifiziertes Segment umschließt, und Bestimmen einer Schnittmenge zwischen mindestens zwei der berechneten 2D-Umrahmungsboxen;
- wenn die Schnittmenge des Paars nicht leer ist, Bestimmen (S420) einer Schnittmenge zwischen den von dem Paar von 3D-Umrahmungsboxen umschlossenen Segmenten, wobei die Bestimmung der Schnittmenge zwischen den Segmenten nur für Segmente durchgeführt wird, für die die Schnittmenge zwischen den 2D-Umrahmungsboxen nicht leer ist;
- wenn die Schnittmenge zwischen den Segmenten nicht leer ist, Zusammenführen (S430) der Segmente durch Berechnen einer resultierenden 3D-Umrahmungsbox, die die Segmente umschließt; und
- Messen (S50) der Größe der Segmente, die in der resultierenden 3D-Umrahmungsbox enthalten sind.

2. Verfahren nach Anspruch 1, ferner umfassend ein iteratives Bestimmen (S40) der Schnittmenge (S410) über Paare von Umrahmungsboxen durch Wiederholen des Bestimmens (S420) und des Zusammenführens (S430) für jedes verbleibende Paar von Umrahmungsboxen.

3. Verfahren nach einem der Ansprüche 1 bis 2, ferner umfassend, wenn die Schnittmenge zwischen den Segmenten leer ist, ein Beibehalten des Paars von Umrahmungsboxen, das jedes Segment umschließt.

4. Verfahren nach einem der Ansprüche 1 bis 3, ferner umfassend ein Erlangen eines Abstands zwischen den Bildern, umfassend die von dem Paar umschlossenen Segmente, wobei die Bestimmung (S420) der Schnittmenge zwischen den von dem Paar umschlossenen Segmenten nur durchgeführt wird, wenn der Abstand zwischen den Bildern, die die Segmente umfasst, unterhalb eines vorbestimmten Schwellenwerts ist.

5. Verfahren nach einem der Ansprüche 1 bis 4, wobei das trainierte neuronale Netz auch zum Ausgeben von Tags konfiguriert ist, die Segmente von menschlichem Körpergewebe identifizieren, und wobei die Bestimmung der Schnittmenge über Paare von Umrahmungsboxen für Segmente mit demselben Tag durchgeführt wird.

6. Verfahren nach einem der Ansprüche 1 bis 5, wobei das Bestimmen der Schnittmenge zwischen den Segmenten Folgendes umfasst:
- Erlangen einer Maske für jedes Segment; und
- Berechnen der Schnittmenge zwischen den bereitgestellten Masken, wobei das Zusammenführen der Segmente nur für Segmente durchgeführt wird, für die die Schnittmenge zwischen den bereitgestellten Masken nicht leer ist.

7. Verfahren nach einem der Ansprüche 2 bis 6, wobei das trainierte neuronale Netz auch zum Ausgeben von 2D-Umrahmungsboxen konfiguriert ist, die die Segmente von menschlichem Körpergewebe umschließen, und wobei ein Berechnen der 2D-Umrahmungsboxen durch das trainierte neuronale Netz durchgeführt wird.

8. Verfahren nach einem der Ansprüche 1 bis 7, wobei ein Messen der Größe der Segmente ferner ein Auswählen des in der resultierenden Umrahmungsbox enthaltenen Segments umfasst, das den längsten Durchmesser aufweist.

9. Verfahren nach einem der Ansprüche 1 bis 8, wobei der Satz von medizinischen Bildern einen Satz von CT-SCAN-Bildern, einen Satz von MRT-Bildern, PET-Scan-Bildern oder Ultraschallbildern umfasst.

10. Verfahren nach einem der Ansprüche 1 bis 9, wobei das menschliche Körpergewebe, das durch den Satz medizinischer Bilder dargestellt ist, einer Läsion in einem Organ oder Gewebe eines Aneurysmas oder eines Organs entspricht.

11. Verfahren zum Identifizieren der Entwicklung eines menschlichen Körpergewebes, umfassend:
- Erlangen eines aktuellen Satzes von medizinischen Bildern, die menschliches Körpergewebe eines Patienten darstellen;
- Verwenden des Verfahrens nach einem der Ansprüche 1 bis 10 zum Erlangen einer aktuellen Messung der Größe der Segmente;
- Erlangen einer Messung, die aus einem vergangenen Satz von medizinischen Bildern erlangt werden, die das menschliche Körpergewebe des Patienten darstellen;
- Berechnen der Differenz zwischen der aktuellen Messung und der vergangenen Messung, wodurch die Entwicklung des menschlichen Körpergewebes identifiziert wird.

12. Computerprogramm, umfassend Anweisungen, die, wenn das Programm von einem Computer ausgeführt wird, den Computer veranlassen, das Verfahren nach einem der Ansprüche 1 bis 10 und das Verfahren nach Anspruch 11 auszuführen.

13. Computerlesbares Speichermedium, auf dem das Computerprogramm nach Anspruch 12 aufgezeichnet ist.

14. System, umfassend einen Prozessor, der mit einem Speicher gekoppelt ist, wobei auf dem Speicher das Computerprogramm nach Anspruch 12 aufgezeichnet ist.

## Revendications

1. Procédé mis en œuvre par ordinateur pour mesurer un tissu corporel humain à partir d'un ensemble d'images médicales représentant le tissu corporel humain, les images étant ordonnées selon un axe de référence, le procédé comprenant :
- l'obtention (S10) d'un réseau de neurones entraîné configuré pour fournir en sortie des segments 2D de tissu corporel humain à partir des images médicales de l'ensemble ;
- l'application (S20) du réseau de neurones entraîné à l'ensemble d'images médicales, identifiant ainsi un ou plusieurs segments 2D de tissu corporel humain pour au moins deux images de l'ensemble ;
- le calcul (S30) d'une boîte englobante 3D entourant chaque segment 2D, la boîte englobante 3D ayant une épaisseur au moins égale à la distance entre deux images consécutives de l'ensemble d'images ;
- la détermination (S410) d'une intersection entre une paire de boîtes englobantes 3D ;
- si l'intersection de la paire est non vide, l'identification des segments compris dans les boîtes englobantes s'intersectant, le calcul de boîtes englobantes 2D pour chaque segment identifié à partir d'au moins deux images de l'ensemble, chaque boîte englobante 2D entourant un segment identifié, et la détermination d'une intersection entre au moins deux des boîtes englobantes 2D calculées ;
- si l'intersection de la paire est non vide, la détermination (S420) d'une intersection entre les segments entourés par la paire de boîtes englobantes 3D ; la détermination de l'intersection entre les segments n'étant effectuée que pour les segments pour lesquels l'intersection entre les boîtes englobantes 2D est non vide ;
- si l'intersection entre les segments est non vide, la fusion (S430) des segments par calcul d'une boîte englobante 3D résultante entourant les segments ; et
- la mesure (S50) de la taille des segments compris dans la boîte englobante 3D résultante.

2. Procédé selon la revendication 1, comprenant en outre la détermination itérative (S40) de l'intersection (S410) sur des paires de boîtes englobantes en répétant la détermination (S420) et la fusion (S430) pour chaque paire restante de boîtes englobantes.

3. Procédé selon l'une quelconque des revendications 1 à 2, comprenant en outre, si l'intersection entre les segments est vide, le maintien de la paire de boîtes englobantes entourant chaque segment.

4. Procédé selon l'une quelconque des revendications 1 à 3, comprenant en outre l'obtention d'une distance entre les images comprenant les segments entourés par la paire, la détermination (S420) de l'intersection entre les segments entourés par la paire n'étant effectuée que lorsque la distance entre les images comprenant les segments est inférieure à un seuil prédéterminé.

5. Procédé selon l'une quelconque des revendications 1 à 4, dans lequel le réseau de neurones entraîné est également configuré pour fournir en sortie des étiquettes identifiant des segments de tissu corporel humain, et dans lequel la détermination de l'intersection sur des paires de boîtes englobantes est effectuée pour des segments ayant la même étiquette.

6. Procédé selon l'une quelconque des revendications 1 à 5, dans lequel la détermination de l'intersection entre les segments comprend :
- l'obtention d'un masque pour chaque segment ; et
- le calcul de l'intersection entre les masques obtenus, la fusion des segments n'étant effectuée que pour les segments pour lesquels l'intersection entre les masques obtenus est non vide.

7. Procédé selon l'une quelconque des revendications 2 à 6, dans lequel le réseau de neurones entraîné est également configuré pour fournir en sortie des boîtes englobantes 2D entourant les segments de tissu corporel humain, et dans lequel le calcul des boîtes englobantes 2D est réalisé par le réseau de neurones entraîné.

8. Procédé selon l'une quelconque des revendications 1 à 7, dans lequel la mesure de la taille des segments comprend en outre la sélection du segment compris dans la boîte englobante résultante ayant le plus long diamètre.

9. Procédé selon l'une quelconque des revendications 1 à 8, dans lequel l'ensemble d'images médicales comprend un ensemble d'images de tomodensitométrie, un ensemble d'images IRM, un ensemble d'images de tomographie par émission de positons (TEP), ou un ensemble d'images échographiques.

10. Procédé selon l'une quelconque des revendications 1 à 9, dans lequel le tissu corporel humain représenté par l'ensemble d'images médicales correspond à une lésion dans un organe, ou à un tissu d'un anévrisme ou à un organe.

11. Procédé d'identification de l'évolution d'un tissu corporel humain, comprenant :
- l'obtention d'un ensemble actuel d'images médicales représentant un tissu corporel humain d'un patient ;
- l'utilisation du procédé selon l'une quelconque des revendications 1 à 10 pour obtenir une mesure actuelle de la taille des segments ;
- l'obtention d'une mesure obtenue à partir d'un ensemble antérieur d'images médicales représentant le tissu corporel humain du patient ;
- le calcul de la différence entre la mesure actuelle et la mesure antérieure, identifiant ainsi l'évolution du tissu corporel humain.

12. Programme informatique comprenant des instructions qui, lorsque le programme est exécuté par un ordinateur, amènent l'ordinateur à mettre en œuvre le procédé selon l'une quelconque des revendications 1 à 10 et le procédé selon la revendication 11.

13. Support de stockage lisible par ordinateur sur lequel est enregistré le programme informatique selon la revendication 12.

14. Système comprenant un processeur couplé à une mémoire, sur laquelle mémoire est enregistré le programme informatique selon la revendication 12.
